# FASCICULE DE BREVET EUROPEEN

(11) **EP 3 047 256 B1**
(45) Date de publication et mention de la délivrance du brevet: **11.04.2018**
(21) Numéro de dépôt: 14790195.3
(22) Date de dépôt: 18.09.2014
(51) Int. Cl.: G01N 21/03, G01N 21/05, G01N 21/3504, G01N 33/497

(54) **CUVE DE MESURE POUR APPAREIL DE MESURE PAR SPECTROMETRIE**
MESSKÜVETTE FÜR EIN SPEKTROMETRISCHES MESSINSTRUMENT
MEASURING CUVETTE FOR SPECTROMETRIC MEASURING INSTRUMENT

(30) Priorité: 18.09.2013 FR 1358974
(43) Date de publication de la demande: 27.07.2016
(73) Titulaire: Olythe, 13857 Aix en Provence (FR)
(72) Inventeur: NESA, Guillaume, F-13090 Aix en Provence (FR); DUBOIS, Grégory, 78600 Maisons-Laffitte (FR); FLESCH, Etienne, F-78570 Andresy (FR); PAFUMI, Lionel, F-13127 Vitrolles (FR); THIERRY, Jean-Pierre, F-78570 Andresy (FR)
(74) Mandataire: Roman, Alexis
(86) Numéro de dépôt international: PCT/FR2014/052320
(87) Numéro de publication internationale: WO 2015/040330

(56) Documents cités:
- EP-A1- 1 306 661
- DE-A1- 3 942 325
- FR-A1- 2 941 530
- GB-A- 2 392 976
- JP-A- 2012 177 690

## Description

### Domaine technique de l'invention.

L'invention a pour objet une cuve de mesure pour appareil de mesure par spectrométrie. Elle a également pour objet un appareil de mesure par spectrométrie intégrant une telle cuve. Elle a encore pour objet un éthylomètre pour mesurer ou détecter un taux de gaz partiel exhalé par un fluide d'haleine et intégrant cette cuve de mesure. Elle a en outre pour objet un procédé de fabrication d'une cuve de mesure dans laquelle est destiné à circuler un gaz à analyser par spectrométrie. Elle a enfin pour objet une méthode pour réaliser une barrière électromagnétique dans une cuve de mesure.

L'invention concerne le domaine technique des éléments constitutifs des appareils de mesure par spectrométrie, et plus particulièrement des éléments constitutif des cuves de mesure. Elle concerne également le domaine technique des dispositifs électroniques portables, tels que les éthylomètres par exemple, pour mesurer ou détecter un taux de gaz partiel exhalé par un fluide d'haleine.

### État de la technique.

On connait par le document brevet FR 2.941.530 (SERES ENVIRONNEMENT), ci-après « document SERES », un éthylomètre comportant un dispositif d'émission d'un rayonnement infrarouge, un récepteur infrarouge et une cuve de mesure dans laquelle circule le fluide d'haleine dont un taux de gaz partiel doit être mesuré ou détecté. La cuve de mesure se présente sous la forme d'un tube creux métallique dont la surface interne est pourvue d'un matériau réfléchissant formant une couche de réflexion optique.

Dans ce type d'appareil, le tube métallique joue un rôle de barrière électromagnétique qui réduit, dans la cuve de mesure, d'éventuels champs électromagnétiques externes (parasites électriques, radiofréquences, ondes électromagnétiques, ...).

Dans la pratique, le tube métallique du document SERES est relativement lourd. En outre, sa surface interne nécessite un traitement de surface spécifique afin de former la couche de réflexion optique. Ce traitement est généralement complexe à réaliser et, en tout état de cause, onéreux.

On connait également par le document brevet EP 1.306.661 (AGILENT TECHNOLOGIES), une cuve de mesure comportant un tube creux similaire au tube du document SERES, et qui présente le même type d'inconvénients.

L'invention vise à remédier à cet état des choses. En particulier, un objectif de l'invention est de proposer une solution alternative pour réduire les effets de champs électromagnétiques externes à l'intérieur de la cuve de mesure, cette solution devant conduire à une cuve qui soit plus légère, moins onéreuse et plus aisée à fabriquer que celle décrite dans le document SERES, tout en gardant la même qualité optique.

Un autre objectif de l'invention est de proposer une cuve de mesure qui soit adaptable à tout type d'appareil de mesure par spectrométrie.

### Divulgation de l'invention.

La solution proposée par l'invention est une cuve de mesure dans laquelle est destiné à circuler un gaz à analyser par spectrométrie. Elle se présente sous la forme d'un tube creux pourvu d'un matériau réfléchissant formant une couche de réflexion optique. Cette cuve est remarquable en ce que :
- le tube creux est réalisé dans un matériau non métallique,
- un article optique souple amovible est appliqué contre la surface interne du tube creux, lequel article comprend un support flexible souple, une face dudit support étant recouverte d'un matériau métallique réfléchissant, ledit article étant inséré dans ledit tube de manière à ce que ledit matériau métallique réfléchissant forme la couche de réflexion optique.

C'est maintenant le matériau métallique réfléchissant de l'article optique souple qui joue le rôle de barrière électromagnétique, et non plus le tube. Ce dernier peut donc être réalisé dans un matériau non métallique, léger et bon marché et sa surface interne ne nécessite pas de traitement de surface particulier contrairement à celui décrit dans le document SERES. En outre, la conception de la cuve de mesure est simple puisque l'article optique est facilement et rapidement insérable dans le tube creux.

D'autres caractéristiques avantageuses de la cuve de mesure objet de l'invention sont listées ci-dessous, chacune de ces caractéristiques pouvant être considérée seule ou en combinaison avec les caractéristiques remarquables définies ci-dessus :
- Le tube creux a préférentiellement une longueur inférieure ou égale à 100 mm, l'article optique ayant une longueur correspondante à celle dudit tube.
- La surface interne du tube creux comporte avantageusement des éléments en relief, l'article optique n'étant en contact avec la surface interne dudit tube qu'au niveau de ces éléments en relief.
- Le support flexible souple est avantageusement réalisé dans un matériau choisi dans le groupe suivant : polyimide, polyépoxyde, polyester, résine époxy renforcée de fibre de verre, substrat d'aluminium.
- Le support flexible souple peut avoir une épaisseur comprise entre 1 µm et 250 µm.
- Le matériau réfléchissant formant la couche de réflexion optique, est avantageusement choisi dans le groupe suivant : or, cobalt, argent, nickel, cuivre, aluminium, chrome, zinc.
- Une couche peut être disposée entre le matériau métallique réfléchissant et la face du support flexible souple, le matériau de cette couche pouvant être choisi dans le groupe suivant : cuivre, aluminium, argent, polyéthylène.

Un autre aspect de l'invention concerne un appareil de mesure par spectrométrie comprenant une cuve de mesure dans laquelle est destiné à circuler un gaz à analyser par spectrométrie, la cuve se présentant sous la forme d'un tube creux pourvu d'un matériau réfléchissant formant une couche de réflexion optique, ladite cuve étant conforme à l'une des caractéristiques précédentes.

Encore un autre aspect de l'invention concerne un éthylomètre pour mesurer le taux de gaz partiel exhalé par un fluide d'haleine, ledit éthylomètre comportant un dispositif d'émission d'un rayonnement infrarouge, un récepteur infrarouge et une cuve de mesure dans laquelle circule le fluide d'haleine, la cuve de mesure se présentant sous la forme d'un tube creux pourvu d'un matériau réfléchissant formant une couche de réflexion optique. Cet éthylomètre est remarquable en ce que :
- le tube creux est réalisé dans un matériau non métallique,
- un article optique souple amovible est appliqué contre la surface interne du tube creux, lequel article comprend un support flexible souple, une face dudit support étant recouverte d'un matériau métallique réfléchissant, ledit article étant positionné dans ledit tube de manière à ce que ledit matériau métallique réfléchissant forme la couche de réflexion optique.

Un aspect supplémentaire de l'invention concerne un procédé de fabrication d'une cuve de mesure dans laquelle est destiné à circuler un gaz à analyser par spectrométrie, la cuve se présentant sous la forme d'un tube creux pourvu d'un matériau réfléchissant formant une couche de réflexion optique. Selon l'invention, ce procédé consiste à :
i) réaliser le tube creux dans un matériau non métallique,
ii) appliquer contre la surface interne du tube creux : un article optique souple amovible, lequel article comprend un support flexible souple, une face dudit support étant recouverte d'un matériau métallique réfléchissant, ledit article étant positionné dans ledit tube de manière à ce que ledit matériau métallique réfléchissant dudit article forme la couche de réflexion optique. Cette étape peut consister à : - rouler ou plier l'article optique; - puis insérer l'article optique ainsi roulé ou plié dans le tube creux de manière à ce que le matériau métallique réfléchissant dudit article forme la couche de réflexion optique.

Un aspect subsidiaire de l'invention concerne une méthode pour réaliser une barrière électromagnétique dans une cuve de mesure dans laquelle est destiné à circuler un gaz à analyser par spectrométrie, la cuve se présentant sous la forme d'un tube creux pourvu d'un matériau réfléchissant formant une couche de réflexion optique, ledit tube étant réalisé dans un matériau non métallique. La méthode consiste à appliquer contre la surface interne du tube creux : un article optique souple amovible, lequel article comprend un support flexible souple, une face dudit support étant recouverte d'un matériau métallique réfléchissant, ledit article étant positionné dans ledit tube de manière à ce que le matériau métallique réfléchissant forme la couche de réflexion optique et forme une barrière électromagnétique entre l'intérieur de la cuve et la surface interne dudit tube.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une vue schématique en coupe d'un article optique conforme à l'invention,
- la figure 2 est une vue schématique de dessus d'un article optique selon la figure 1,
- la figure 3 est une vue schématique en coupe longitudinale, d'un éthylomètre conforme à l'invention,
- la figure 4 est une vue en coupe selon A-A de l'éthylomètre de la figure 3.

### Modes préférés de réalisation de l'invention.

La cuve de mesure objet de l'invention est particulièrement, mais non exclusivement, destinée à être utilisée dans un appareil de mesure par spectrométrie. Elle est notamment conçue pour être intégrée dans un éthylomètre, mais peut également être intégrée dans tout autre appareil qui mesure un paramètre (concentration d'alcool, de CO, de CO2, de H₂O, ...) dans un fluide d'haleine ou dans tout autre fluide (par exemple une vapeur ou un gaz d'échappement).

Dans un souci de clarté et de concision, la suite de la description fait uniquement référence à un éthylomètre, sans que cela puisse être considéré comme une limitation de la protection recherchée. Par « éthylomètre », on entend au sens de la présente invention tout appareil (y compris les éthylotests) permettant de mesurer ou détecter un taux de gaz partiel exhalé par un fluide d'haleine, et en particulier de mesurer la concentration d'alcool dans l'air expiré et/ou de détecter un seuil de concentration d'alcool dans l'air expiré.

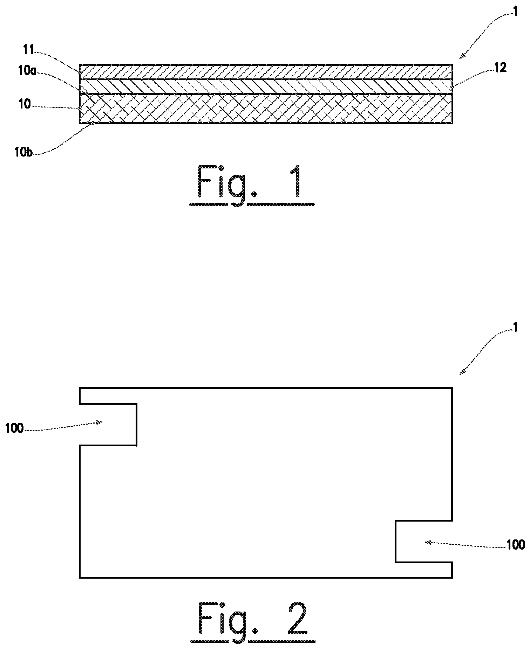

La figure 1 illustre un article optique souple servant à former la cuve de mesure. Cet article 1 comprend un support flexible souple 10 consiste en un film mince ayant une épaisseur comprise entre 1 µm et 250 µm, préférentiellement environ 25 µm. Un bon rapport souplesse/résistance est obtenu avec ces valeurs d'épaisseur. Sa longueur et sa largeur dépendent des dimensions de la cuve de mesure dans laquelle il est intégré. Le support 10 est avantageusement réalisé dans un matériau choisi dans le groupe suivant : polyimide (par ex : Kapton®), polyépoxyde, polyester, résine époxy renforcée de fibre de verre, substrat d'aluminium (par ex : support COOL-CLAD® commercialisé par la société AI TECHNOLOGY). Tout autre matériau généralement utilisé pour la fabrication de circuit imprimé souple peut toutefois être envisagé. Le support 10 peut être obtenu par moulage, extrusion, laminage, etc.

Le support 10 comprend une face supérieure 10a et une face inférieure 10b qui sont opposées. Sur la figure 1 annexée, la face supérieure 10a est recouverte d'un matériau métallique réfléchissant 11 pour former une couche de réflexion optique sur laquelle rebondira le rayonnement infrarouge. On peut toutefois prévoir que le matériau métallique réfléchissant 11 soit déposé sur la face inférieure 10b. Pour que la couche de réflexion soit la plus réfléchissante possible et afin de limiter les pertes énergétiques du rayonnement émis, le matériau métallique réfléchissant 11 est préférentiellement choisi dans le groupe suivant: or, cobalt, argent, nickel, cuivre, aluminium, chrome, zinc.

Le matériau métallique réfléchissant 11 présente une épaisseur comprise entre 0,01 µm et 500 µm. Il peut être déposé par collage, par dépôt électrochimique, par dépôt électrolytique, par impression, par sérigraphie, chauffage, ou par tout autre procédé d'adhésion de couche fine.

Dans le but d'assurer un bon maintien en position du matériau métallique réfléchissant 11 sur la face supérieure 10a du support 10, une couche d'accroche 12 peut être déposée sur cette face. Cette couche 12 est par exemple constituée d'une couche de cuivre, d'aluminium, d'argent, ou de polyéthylène, dont l'épaisseur est par exemple comprise entre 0,1 µm et 500 µm, déposée par un procédé d'adhésion de couche fine du type mentionné au paragraphe précédent. La couche 12 n'est pas indispensable et peut notamment être évitée dans le cas où le matériau métallique réfléchissant 11 est déposé, par exemple, par dépôt électrolytique.

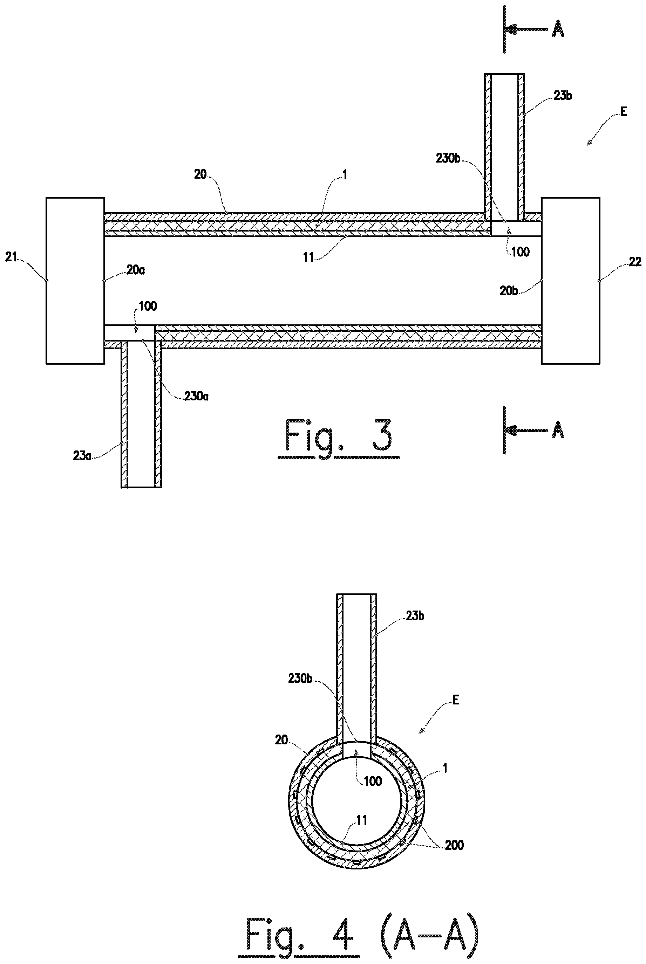

L'intégration de l'article 1 dans la cuve de mesure d'un éthylomètre portable, va maintenant être détaillée en référence aux figures 3 et 4. Cet éthylomètre E est du type décrit dans le document SERES précité. La cuve de mesure se présentant sous la forme d'un tube creux 20. Ce dernier est typiquement de section circulaire mais peut être de section carrée, rectangulaire, ovale, etc. Le tube 20 est réalisé dans un matériau non métallique, par exemple en plastique (ex : PVC, ABS), en carbone, en matériau composite, etc. Il peut être obtenu par moulage, par extrusion ou par tout autre procédé convenant à l'homme du métier. Sa surface interne ne nécessite pas de traitement de surface particulier contrairement à celui décrit dans le document SERES.

Selon un mode préféré de réalisation, la longueur du tube 20 est comprise entre 5 mm et 200 mm, préférentiellement inférieure ou égale à 100 mm, l'invention permettant d'utiliser une cuve de mesure plus courte que celle du document SERES. Son diamètre interne est inférieur à 15 mm, par exemple compris entre 4 mm et 15 mm. Et son épaisseur est inférieure à 5 mm, par exemple comprise entre 1 mm et 5 mm.

Une extrémité 20a du tube 20 est pourvue d'un dispositif 21 d'émission d'un rayonnement infrarouge, avantageusement dans des longueurs d'ondes comprises entre 1 µm et 12 µm. L'autre extrémité 20b est pourvue d'un récepteur infrarouge 22. L'émetteur 21 et le récepteur 22 infrarouge sont du type connu de l'homme du métier. Le fluide d'haleine circule dans la cuve de mesure entre les deux extrémités 20a, 20b du tube 20. Plus particulièrement, le fluide pénètre dans le tube 20 par l'intermédiaire d'une tubulure d'entrée 23a (dans laquelle souffle l'utilisateur) installée au niveau de l'extrémité 20a, et ressort dudit tube par l'intermédiaire d'une tubulure de sortie 23b installée au niveau de l'extrémité opposée 23b. Les deux tubulures 23a et 23b peuvent être situées du même côté du tube 20, ou au contraire sur deux côtés opposés (figure 3). Un système de pompage peut être associé aux tubulures 23a et 23b afin d'assurer la circulation de l'échantillon de fluide soufflé.

L'article 1 est inséré dans le tube 20 de manière à ce que le matériau métallique réfléchissant 11 forme la couche de réflexion optique. Lorsque le tube 20 est de section circulaire, l'article 1 est roulé, manuellement ou automatiquement, de manière à former un cylindre. Dans le cas où le tube 20 n'est pas de section circulaire, mais de section carrée, rectangulaire ou d'une autre forme polygonale, l'article 1 est plié de manière à former un tube ayant cette section particulière. Le matériau métallique réfléchissant 11 forme la surface interne de ce cylindre (ou de ce tube). Cette disposition permet d'optimiser les longueurs des trajets optiques dans le tube 20, tout en conservant une quantité de lumière suffisante jusqu'au récepteur 22. Il en résulte que la cuve de mesure peut être plus courte que celle de l'éthylomètre décrit dans le document SERES.

L'article optique 1 a une longueur correspondante à celle du tube 20 de sorte que la surface interne de ce dernier soit totalement, ou sensiblement totalement, recouverte par ledit article. En effet, certaines zones de la surface interne du tube 20 peuvent ne pas être recouvertes, notamment au niveau des extrémités 20a et 20b, tout en préservant une qualité de mesure acceptable.

L'article 1 ainsi conformé est alors inséré dans le tube 20, au niveau d'une des extrémités 20a ou 20b, de sorte que le matériau métallique réfléchissant 11 forme la couche de réflexion optique contre lequel rebondira le rayonnement infrarouge. Dans la configuration de la figure 1, c'est la face inférieure 10b du support 10 qui est en contact avec la surface interne du tube 20.

Ainsi positionné entre la surface interne du tube 20 et l'intérieur de la cuve de mesure, l'article optique 1 - et plus précisément le matériau métallique réfléchissant 11 - forme une barrière électromagnétique. Tout ou parties des champs électromagnétiques externes, susceptibles de pénétrer à l'intérieur de la cuve de mesure et de perturber le récepteur 22 infrarouge - et donc la précisions des mesures -, sont de fait stoppées par le matériau métallique réfléchissant 11.

Lorsque l'article 1 est mis en forme, il a naturellement tendance à se dérouler (ou se déplier) pour retrouver sa forme plate d'origine. Il résulte de cette aptitude que l'article 1 est naturellement maintenu en position à l'intérieur du tube 20, sans qu'il soit nécessaire de prévoir un autre système de fixation mécanique ou par colle. Un tel système peut toutefois être envisagé par mesure de précaution.

La surface interne du tube 20 peut comporter des éléments en relief 200. Ces derniers consistent par exemple en des nervures longitudinales ou radiales, ou selon toute autre forme présentant des creux et des bosses sur la surface interne du tube 20. Comme cela apparait sur la figure 4, lorsque l'article optique 1 est inséré dans le tube 20, il n'est en contact qu'avec ces éléments en relief 200.

Une fois que l'article 1 est mis en forme et installé dans le tube 20, et que la cuve de mesure est ainsi fabriquée, les autres composants 21, 22, 23a, 23b sont mis en place.

Concernant les tubulures 23a, 23b, il est nécessaire qu'elles débouchent à l'intérieur de la cuve de mesure, malgré la présence de l'article 1 qui recouvre la surface interne du tube 20. Pour ce faire, et comme cela apparait sur la figure 2, l'article 1 comprend des encoches, des perçages, ou, de manière plus générale, des évidements 100, dont les dimensions sont ajustées aux diamètres des tubulures 23a, 23b. Ces évidements 100 sont situés au niveau des bords latéraux de l'article 1. Lorsque l'article 1 est mis en forme et inséré dans le tube 10, les évidements 100 sont placés en vis-à-vis des extrémités débouchantes 230a, 230b des tubulures 23a, 23b et laissent libres ces dernières.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de la portée de l'invention. En particulier, La cuve de mesure peut être utilisée pour traiter des signaux autres que des signaux infrarouges et/ou avec une technique d'analyse autre que la spectrométrie.

## Revendications

1. Cuve de mesure dans laquelle est destiné à circuler un gaz à analyser par spectrométrie, la cuve se présentant sous la forme d'un tube creux (20) pourvu d'un matériau réfléchissant formant une couche de réflexion optique, **se caractérisant par le fait que** :
- le tube creux (20) est réalisé dans un matériau non métallique,
- un article optique souple (1) amovible est appliqué contre la surface interne du tube creux (20), lequel article comprend un support flexible souple (10), une face (10a) dudit support étant recouverte d'un matériau métallique réfléchissant (11), ledit article étant inséré dans ledit tube de manière à ce que ledit matériau métallique réfléchissant forme la couche de réflexion optique.

2. Cuve selon la revendication 1, dans laquelle le tube creux (20) a une longueur inférieure ou égale à 100 mm, l'article optique (1) ayant une longueur correspondante à celle dudit tube.

3. Cuve selon l'une des revendications 1 ou 2, dans laquelle la surface interne du tube creux (20) comporte des éléments en relief (200), l'article optique (1) n'étant en contact avec la surface interne dudit tube qu'au niveau de ces éléments en relief.

4. Cuve selon l'une des revendications 1 à 3, dans laquelle le support flexible souple (10) est réalisé dans un matériau choisi dans le groupe suivant : polyimide, polyépoxyde, polyester, résine époxy renforcée de fibre de verre, substrat d'aluminium.

5. Cuve selon l'une des revendications 1 à 4, dans laquelle le support flexible souple (10) a une épaisseur comprise entre 1 µm et 250 µm.

6. Cuve selon l'une des revendications 1 à 5, dans laquelle le matériau métallique réfléchissant (11) formant la couche de réflexion optique, est choisi dans le groupe suivant : or, cobalt, argent, nickel, cuivre, aluminium, chrome, zinc.

7. Cuve selon l'une des revendications 1 à 6, dans laquelle une couche (12) est disposée entre le matériau métallique réfléchissant (11) et la face (10a) du support flexible souple (10).

8. Cuve selon la revendication 7, dans laquelle le matériau de la couche (12) est choisi dans le groupe suivant : cuivre, aluminium, argent, polyéthylène.

9. Appareil de mesure par spectrométrie comprenant une cuve de mesure dans laquelle est destiné à circuler un gaz à analyser par spectrométrie, la cuve se présentant sous la forme d'un tube creux (20) pourvu d'un matériau réfléchissant formant une couche de réflexion optique, **se caractérisant par le fait que** ladite cuve est conforme à l'une des revendications 1 à 8.

10. Ethylomètre pour mesurer le taux de gaz partiel exhalé par un fluide d'haleine, ledit éthylomètre comportant un dispositif d'émission d'un rayonnement infrarouge (21), un récepteur infrarouge (22) et une cuve de mesure dans laquelle circule le fluide d'haleine, la cuve de mesure se présentant sous la forme d'un tube creux (20) pourvu d'un matériau réfléchissant formant une couche de réflexion optique, **se caractérisant par le fait que** ladite cuve est conforme à l'une des revendications 1 à 8.

11. Procédé de fabrication d'une cuve de mesure dans laquelle est destiné à circuler un gaz à analyser par spectrométrie, la cuve se présentant sous la forme d'un tube creux (20) pourvu d'un matériau réfléchissant formant une couche de réflexion optique, **se caractérisant par le fait que** le procédé consiste à :
i) réaliser le tube creux (20) dans un matériau non métallique,
ii) appliquer contre la surface interne du tube creux (20) : un article optique souple (1) amovible, lequel article comprend un support flexible souple (10), une face (10a) dudit support étant recouverte d'un matériau métallique réfléchissant (11), ledit article étant positionné dans ledit tube de manière à ce que ledit matériau métallique réfléchissant forme la couche de réflexion optique.

12. Procédé selon la revendication 11, dans lequel l'étape ii) consiste à :
- rouler ou plier l'article optique (1),
- insérer l'article optique (1) ainsi roulé ou plié dans le tube creux (20) de manière à ce que le matériau métallique réfléchissant (11) dudit article forme la couche de réflexion optique.

13. Méthode pour réaliser une barrière électromagnétique dans une cuve de mesure dans laquelle est destiné à circuler un gaz à analyser par spectrométrie, la cuve se présentant sous la forme d'un tube creux (20) pourvu d'un matériau réfléchissant formant une couche de réflexion optique, ledit tube étant réalisé dans un matériau non métallique, la méthode consistant à appliquer contre la surface interne du tube creux: un article optique souple amovible (1), lequel article comprend un support flexible souple (10), une face (10a) dudit support étant recouverte d'un matériau métallique réfléchissant (11), ledit article étant positionné dans ledit tube de manière à ce que ledit matériau métallique réfléchissant forme la couche de réflexion optique et forme une barrière électromagnétique entre l'intérieur de la cuve et la surface interne dudit tube.

## Patentansprüche

1. Messküvette, in der ein durch Spektrometrie zu analysierendes Gas zirkulieren soll, wobei die Küvette in Form eines hohlen Rohres (20) vorliegt, das mit einem reflektierenden Material versehen ist, das eine optische Reflexionsschicht bildet, **dadurch gekennzeichnet, dass**:
- das hohle Rohr (20) aus einem nichtmetallischen Material hergestellt ist,
- ein abnehmbarer weicher optischer Gegenstand (1) an die Innenfläche des hohlen Rohres (20) angelegt wird, wobei der Gegenstand eine biegsame weiche Auflage (10) umfasst, wobei eine Fläche (10a) der Auflage mit einem reflektierenden metallischen Material (11) beschichtet ist, wobei der Gegenstand derart in das Rohr eingefügt ist, dass das reflektierende metallische Material die optische Reflexionsschicht bildet.

2. Küvette nach Anspruch 1, wobei das hohle Rohr (20) eine Länge von kleiner oder gleich 100 mm aufweist, wobei der optische Gegenstand (1) eine Länge aufweist, die jener des Rohres entspricht.

3. Küvette nach einem der Ansprüche 1 oder 2, wobei die Innenfläche des hohlen Rohres (20) erhabene Elemente (200) aufweist, wobei der optische Gegenstand (1) nur im Bereich dieser erhabenen Elemente in Kontakt mit der Innenfläche des Rohres ist.

4. Küvette nach einem der Ansprüche 1 bis 3, wobei die biegsame weiche Auflage (10) aus einem Material hergestellt ist, das aus der folgenden Gruppe ausgewählt ist: Polyimid, Polyepoxid, Polyester, glasfaserverstärktes Epoxidharz, Aluminiumsubstrat.

5. Küvette nach einem der Ansprüche 1 bis 4, wobei die biegsame weiche Auflage (10) eine Stärke zwischen 1 µm und 250 µm aufweist.

6. Küvette nach einem der Ansprüche 1 bis 5, wobei das reflektierende metallische Material (11), das die optische Reflexionsschicht bildet, aus der folgenden Gruppe ausgewählt ist: Gold, Kobalt, Silber, Nickel, Kupfer, Aluminium, Chrom, Zink.

7. Küvette nach einem der Ansprüche 1 bis 6, wobei eine Schicht (12) zwischen dem reflektierenden metallischen Material (11) und der Fläche (10a) der biegsamen weichen Auflage (10) angeordnet ist.

8. Küvette nach Anspruch 7, wobei das Material der Schicht (12) aus der folgenden Gruppe ausgewählt ist: Kupfer, Aluminium, Silber, Polyethylen.

9. Spektrometrisches Messgerät, umfassend eine Messküvette, in der ein durch Spektrometrie zu analysierendes Gas zirkulieren soll, wobei die Küvette in Form eines hohlen Rohres (20) vorliegt, das mit einem reflektierenden Material versehen ist, das eine optische Reflexionsschicht bildet, **dadurch gekennzeichnet, dass** die Küvette einem der Ansprüche 1 bis 8 entspricht.

10. Alkoholmessgerät zum Messen des in einem Atemfluid ausgeatmeten partiellen Gasanteils, wobei das Alkoholmessgerät eine Vorrichtung zur Aussendung von Infrarotstrahlung (21), einen Infrarotempfänger (22) und eine Messküvette umfasst, in der das Atemfluid zirkuliert, wobei die Messküvette in Form eines hohlen Rohres (20) vorliegt, das mit einem reflektierenden Material versehen ist, das eine optische Reflexionsschicht bildet, **dadurch gekennzeichnet, dass** die Küvette einem der Ansprüche 1 bis 8 entspricht.

11. Verfahren zur Herstellung einer Messküvette, in der ein durch Spektrometrie zu analysierendes Gas zirkulieren soll, wobei die Küvette in Form eines hohlen Rohres (20) vorliegt, das mit einem reflektierenden Material versehen ist, das eine optische Reflexionsschicht bildet, **dadurch gekennzeichnet, dass** das Verfahren darin besteht:
i) das hohle Rohr (20) aus einem nichtmetallischen Material herzustellen,
ii) einen abnehmbaren weichen optischen Gegenstand (1) an die Innenfläche des hohlen Rohres (20) anzulegen, wobei dieser Gegenstand eine biegsame weiche Auflage (10) umfasst, wobei eine Fläche (10a) der Auflage mit einem reflektierenden metallischen Material (11) beschichtet ist, wobei der Gegenstand derart in dem Rohr positioniert wird, dass das reflektierende metallische Material die optische Reflexionsschicht bildet.

12. Verfahren nach Anspruch 11, wobei der Schritt ii) darin besteht:
- den optischen Gegenstand (1) zu rollen oder zu biegen,
- den auf diese Weise gerollten oder gebogenen optischen Gegenstand (1) derart in das hohle Rohr (20) einzufügen, dass das reflektierende metallische Material (11) des Gegenstands die optische Reflexionsschicht bildet.

13. Verfahren zur Herstellung einer elektromagnetischen Abschirmung in einer Messküvette, in der ein durch Spektrometrie zu analysierendes Gas zirkulieren soll, wobei die Küvette in Form eines hohlen Rohres (20) vorliegt, das mit einem reflektierenden Material versehen ist, das eine optische Reflexionsschicht bildet, wobei das Rohr aus einem nichtmetallischen Material hergestellt ist, wobei das Verfahren darin besteht, an die Innenfläche des hohlen Rohres einen abnehmbaren weichen optischen Gegenstand (1) anzulegen, wobei der Gegenstand eine biegsame weiche Auflage (10) umfasst, wobei eine Fläche (10a) der Auflage mit einem reflektierenden metallischen Material (11) beschichtet ist, wobei der Gegenstand derart in dem Rohr positioniert wird, dass das reflektierende metallische Material die optische Reflexionsschicht bildet und eine elektromagnetische Abschirmung zwischen dem Inneren der Küvette und der Innenfläche des Rohres bildet.

## Claims

1. Measurement chamber through which a gas to be analysed by spectroscopy is intended to flow, the chamber taking the form of a hollow tube (20) provided with a reflective material forming a reflective optical layer, **characterized in that:**
- the hollow tube (20) is made from a non-metal; and
- a supple removable optical article (1) is applied to the internal surface of the hollow tube (20), said article comprising a supple flexible carrier (10), a face (10a) of said carrier being covered with a reflective metal (11), said article being inserted into said tube so that said reflective metal forms the reflective optical layer.

2. Chamber according to Claim 1, wherein the hollow tube (20) has a length smaller than or equal to 100 mm, the optical article (1) having a length corresponding to that of said tube.

3. Chamber according to either of Claims 1 and 2, wherein the internal surface of the hollow tube (20) includes relief elements (200), the optical article (1) making contact with the internal surface of said tube only level with these relief elements.

4. Chamber according to one of Claims 1 to 3, wherein the supple flexible carrier (10) is made from a material chosen from the following group: polyimide, polyepoxide, polyester, glass-fibre-reinforced epoxy resin, aluminium substrate.

5. Chamber according to one of Claims 1 to 4, wherein the supple flexible carrier (10) has a thickness comprised between 1 µm and 250 µm.

6. Chamber according to one of Claims 1 to 5, wherein the reflective metal (11) forming the reflective optical layer is chosen from the following group: gold, cobalt, silver, nickel, copper, aluminium, chromium, zinc.

7. Chamber according to one of Claims 1 to 6, wherein a layer (12) is placed between the reflective metal (11) and the face (10a) of the supple flexible carrier (10).

8. Chamber according to Claim 7, wherein the material of the layer (12) is chosen from the following group: copper, aluminium, silver, polyethylene.

9. Spectroscopic measuring apparatus comprising a measurement chamber through which a gas to be analysed by spectroscopy is intended to flow, the chamber taking the form of a hollow tube (20) provided with a reflective material forming a reflective optical layer, **characterized in that** said chamber is according to one of Claims 1 to 8.

10. Breathalyser for measuring the content of a constituent gas exhaled in a breath fluid, said breathalyser including a device for emitting infrared radiation (21), an infrared receiver (22) and a measurement chamber through which the breath fluid flows, the measurement chamber taking the form of a hollow tube (20) provided with a reflective material forming a reflective optical layer, **characterized in that** said chamber is according to one of Claims 1 to 8.

11. Process for manufacturing a measurement chamber through which a gas to be analysed by spectroscopy is intended to flow, the chamber taking the form of a hollow tube (20) provided with a reflective material forming a reflective optical layer, **characterized in that** the process consists in:
i) making the hollow tube (20) from a non-metal; and
ii) applying to the internal surface of the hollow tube (20): a supple removable optical article (1), said article comprising a supple flexible carrier (10), a face (10a) of said carrier being covered with a reflective metal (11), said article being positioned in said tube so that said reflective metal forms the reflective optical layer.

12. Process according to Claim 11, wherein step ii) consists in:
- rolling or folding the optical article (1); and
- inserting the optical article (1) thus rolled or folded into the hollow tube (20) so that the reflective metal (11) of said article forms the reflective optical layer.

13. Method for producing an electromagnetic barrier in a measurement chamber through which a gas to be analysed by spectroscopy is intended to flow, the chamber taking the form of a hollow tube (20) provided with a reflective material forming a reflective optical layer, said tube being made from a non-metal, the method consisting in applying to the internal surface of the hollow tube: a supple removable optical article (1), said article comprising a supple flexible carrier (10), a face (10a) of said carrier being covered with a reflective metal (11), said article being positioned in said tube so that said reflective metal forms the reflective optical layer and forms an electromagnetic barrier between the interior of the chamber and the internal surface of said tube.
